Europäisches Patentamt

European Patent Office

Office européen des brevets

(19)

(11) Veröffentlichungsnummer: **0 216 334**
**B1**

(12) EUROPÄISCHE PATENTSCHRIFT

(45) Veröffentlichungstag der Patentschrift:
**31.10.90**

(51) Int. Cl.⁵: **A61K 7/13**

(21) Anmeldenummer: **86112978.1**

(22) Anmeldetag: **19.09.86**

(54) Oxidationshaarfärbezubereitung.

(30) Priorität: **27.09.85 DE 3534471**

(43) Veröffentlichungstag der Anmeldung:
**01.04.87 Patentblatt 87/14**

(45) Bekanntmachung des Hinweises auf die Patenterteilung:
**31.10.90 Patentblatt 90/44**

(84) Benannte Vertragsstaaten:
**AT BE CH DE FR GB IT LI NL SE**

(56) Entgegenhaltungen:
**DE-A- 1 942 570**
**FR-A- 2 532 174**
**US-A- 4 247 001**

**CHEMICAL ABSTRACTS, Band 98, Nr. 22, Mai 1983,
Seite 359, Zusammenfassung Nr. 185396y, Columbus,
Ohio, US; & JP-A-58 32 815**

(73) Patentinhaber: **Henkel Kommanditgesellschaft auf
Aktien, Postfach 1100 Henkelstrasse 67,
D-4000 Düsseldorf-Holthausen(DE)**

(72) Erfinder: **Hollenberg, Detlef, Dr., Hugo-Wolff-Strasse 15,
D-4010 Hilden(DE)**
Erfinder: **Neuhaus, Winifried, Stormstrasse 2,
D-4020 Mettmann 2(DE)**
Erfinder: **Schrader, Dieter, Itterstrasse 7,
D-4000 Düsseldorf 13(DE)**

## Beschreibung

Gegenstand der Erfindung ist eine Oxidationshaarfärbezubereitung, bestehend aus einem Zweikammer-Misch- und Abgabebehälter und den darin verpackten Komponenten, bei der eine besonders leichte und störungsfreie Vermischung der Komponenten vor der Anwendung auf dem Haar erreicht wird.

Für das Färben von Haaren spielen die sogenannten Oxidationsfarben, die aus Oxidationsfarbstoffvorprodukten durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate, 4-Aminopyrazolonderivate und Tetraaminopyrimidine eingesetzt. Als sogenannte Kupplersubstanzen werden m-Phenylendiaminderivate, Phenole, Naphtole, Resorcinderivate und Pyrazolone verwendet.

Zur Herstellung von Haarfärbemitteln werden die Oxidationsfarbstoffvorprodukte in einer Menge von etwa 0,1 bis 5,0 Gew.-% in einen geeigneten kosmetischen Träger eingearbeitet, z. B. in ein Gel oder in eine Creme.

Vor der Anwendung wird das Oxidationshaarfärbegel oder die Oxidationshaarfärbecreme mit einer Oxidationsmittelzubereitung, z. B. einer $H_2O_2$-Lösung, die ein Verdickungsmittel enthält, vereinigt, so daß sich eine verdickte Haarfärbezubereitung bildet, in der die oxidative Entwicklung der Farbstoffe erfolgen kann und die in dieser Form auf das zu färbende Haar aufgetragen wird.

Für die Heimanwendung wurden Oxidationshaarfärbezubereitungen eingeführt, die aus einem Zweikammer-Misch- und Abgabebehälter und darin getrennt voneinander enthaltenen Komponenten, einer Oxidationshaarfärbecreme (A) und einer Oxidationsmittelzubereitung (B), bestehen. Solche Applikationsbehälter sind so ausgebildet, daß die Trennwand zwischen den beiden Kammern von außen mechanisch zerstörbar ist und daß eine der Kammern eine Abgabeöffnung besitzt. Kurz vor der Anwendung wird die Trennwand zerstört, worauf die Komponenten A und B vereinigt und durch Schütteln oder andere mechanische Maßnahmen vermischt werden. Dann kann die gebrauchsfertige Haarfärbezubereitung durch die Abgabeöffnung entnommen bzw. direkt auf das zu färbende Haar aufgetragen werden.

Ein Problem bei dieser Art der Zweikammer-Oxidationshaarfärbezubereitungen besteht nun darin, daß die Durchmischung der relativ hochviskosen Färbecreme mit der relativ dünnflüssigen $H_2O_2$-Zubereitung erschwert ist und auch durch längeres Schütteln des Behälters oft keine homogene Mischung erreicht wird. Eine Lösung des Problems durch Senkung der Viskosität der Oxidationsfärbecreme ist nicht gangbar, da die gebrauchsfertige Haarfärbezubereitung eine gewisse Zähigkeit aufweisen muß, damit sie nicht von dem zu färbenden Haar abläuft. Auch durch einen erhöhten Einsatz an Verdickungsmittel in der Oxidationsmittelzubereitung (B) kann diesem Problem nicht befriedigend begegnet werden, da die hierfür bekannten Verdickungsmittel vom Typ der carboxylgruppenhaltigen Polymeren, speziell Acrylsäure- und Methacrylsäurepolymerisate, im schwach sauren Milieu der Oxidationsmittelzubereitung als dünnflüssige Dispersionen vorliegen, bei der Vereinigung mit der alkalischen Färbecreme aber gelieren und im Grenzphasenbereich sich dann schwer auflösbare Gelaggregate bilden.

Aus FR-A 2 532 174 war eine Oxidationsfärbecreme in Form einer Öl-in-Wasser-Emulsion bekannt, die durch Schütteln in einer Applikationsflasche mit einer nicht emulsionsförmigen Oxidationsmittelzubereitung vermischbar ist. Aus DE-A 1 942 570 war eine Oxidationsmittelzubereitung in Form einer Öl-in-Wasser-Emulsion bekannt, die mit einer nicht emulsionsförmigen Farbstoffzubereitung gemeinsam unter Druck aus einem Zweikammerbehälter appliziert werden soll.

Es wurde nun gefunden, daß sich das Problem auf verblüffend einfache Weise lösen läßt, wenn man der Oxidationsmittelzubeitung kein Verdickungsmittel vom Typ der in Wasser oder wäßrigen alkalilöslichen Polymeren mehr zusetzt, sondern sowohl die Oxidationshaarfärbecreme (A) als auch die Oxidationsmittelzubereitung (B) als Öl-in-Wasser-Emulsion formuliert und die Viskosität dieser Emulsionen und ihr Mischungsverhältnis so aufeinander abstimmt, daß sich nach dem Vermischen ein gebrauchsfertiges Haarfärbemittel mit einer Viskosität bei 20°C von 5 bis 15 Pa • s bei einer Scherspannung von D = 3 bis 6 $sec^{-1}$ bildet. Das Mischungsverhältnis der Komponenten A und B liegt zweckmäßigerweise in einem Bereich von A : B = 3 : 1 bis 1 : 1.

Gegenstand der Erfindung ist demnach eine Oxidationshaarfärbezubereitung, bestehend aus einem Zweikammer-Misch- und Abgabebehälter und den darin verpackten Komponenten, wobei der Behälter so ausgebildet ist, daß in den zwei durch eine mechanisch zerstörbare Trennwand getrennten Kammern eine Oxidationshaarfärbecreme (A) und eine Oxidationsmittelzubereitung (B) isoliert voneinander verpackt sind und durch mechanische Zerstörung der Trennwand eins Vereinigung und Durchmischung der beiden Komponenten vor der Abgabe aus der an einer der beiden Kammern befindlichen Abgabeöffnung möglich ist, dadurch gekennzeichnet, daß

– die Oxidationshaarfärbecreme (A) in Form einer Öl-in-Wasser-Emulsion mit einem Gehalt von 0,1 bis 5,0 Gew.-% an Oxidationshaarfarbstoffvorprodukten und
– die Oxidationsmittelzubereitung (B) in Form einer Öl-in-Wasser-Emulsion mit einem Gehalt von 1,5 bis 15 Gew.-% $H_2O_2$ vorliegt.

und die Komponenten A und B im Gewichtsverhältnis A : B = 3 : 1 bis 1 : 1 vorliegen und nach dem Vermischen ein Haarfärbemittel mit einer Viskosität bei 20 °C von 5 bis 15 Pa.s bei einer Scherspannung von D = 3 bis 6 sec$^{-1}$ ergeben.

Zweikammer-Misch- und Abgabebehälter, die sich für die Ausführung der Erfindung eignen, sind z. B. aus US-PS 4 103 772, US-PS 4 174 035 und US-PS 4 247 001 bekannt und in ähnlichen Ausführungsformen auf dem Markt.

Die Oxidationshaarfärbecreme (A) und die Oxidationsmittelzubereitung (B) stellen erfindungsgemäß Öl-in-Wasser-Emulsionen mit einer diskontinuierlichen Öl- oder Fettphase und einer kontinuierlichen wäßrigen Phase dar. Die Viskosität dieser O/W-Emulsionen läßt sich durch das Verhältnis von Ölphase zu Wasserphase und durch die Art der verwendeten Fettkomponenten in weiten Grenzen so steuern, daß beim Vermischen der Komponenten sich die erfindungsgemäße Viskosität des Haarfärbemittels ergibt. Es ist jedoch für die Ausführung der Erifndung bevorzugt, daß die Oxidationshaarfärbecreme (A) eine Viskosität von ca. 5 bis 20 Pa.s und die Oxidationsmittelzubereitung eine Viskosität von 0,5 bis 5 Pa.s, jeweils gemessen bei 20 °C und bei einer Scherspannung von D = 3 bis 6 sec$^{-1}$ aufweisen.

Als Öl- oder Fettkomponenten können Paraffine, Vaseline, Wachse, Hartfette, kosmetische Ölkomponenten und Fettalkohole eingesetzt werden. Als Emulgatoren können anionische, nichtionogene zwitterionische und amphotere Tenside verwendet werden.

Anionische Tenside, die sich als Emulgatoren eignen, sind gekennzeichnet durch eine bevorzugt lineare Alkylgruppe mit 12 bis 18 C-Atomen und eine daran gebundene anionische Gruppe, z. B. eine -COO$^{(-)}$, -SO$_3$$^{(-)}$ oder -O-(CH$_2$-CH$_2$O)$_x$-SO$_3$$^{(-)}$-Gruppe, in der x = 0 oder eine Zahl bis 50 bedeutet. Nichtionische Tenside, die sich als Emulgatoren eignen, sind z. B. Anlagerungsprodukte von 2 bis 30 Mol Ethylenoxid an Fettalkohole mit 12 bis 18 C-Atomen, an Alkylphenole mit 8 bis 15 C-Atomen in der Alkylgruppe, an Fettsäuren mit 12 bis 18 C-Atomen, an Glycerin-Fettsäure(C$_{12}$-C$_{18}$)-partialester, an Sorbitan-Fettsäure(C$_{12}$-C$_{18}$)-partialester, an Fettamine mit 12 bis 18 C-Atomen, an Fettsäure(C$_{12}$-C$_{18}$)-monoethanolamid oder an Alkyl(C$_{12}$-C$_{18}$)glucoside.

Das Mengenverhältnis von Fettkomponenten zu Emulgatoren kann in einem weiten Bereich von 1 : 0,5 bis 1 : 5 schwanken. Die für eine bestimmte Fettkomponente am besten geeignete Art und Menge an Emulgatoren läßt sich durch einfache Versuche ermitteln. Bevorzugt wird als Fettkomponente ein gesättigter Fettalkohol oder Fettalkoholschnitt mit 12 bis 22 C-Atomen und als Emulgator ein Alkyl- und/oder Alkylpolyglycolethersulfat der allgemeinen Formel I

R$^1$ - O (CH$_2$CH$_2$O)$_x$ - SO$_3$M    (I)

verwendet, in der R$^1$ eine Alkylgruppe mit 8 bis 18 C-Atomen, M ein Alkali-, Magnesium-, Ammoniumoder ein Mono-, Di- oder Trialkanolammoniumion mit 2 bis 4 C-Atomen in der Alkanolgruppe ist und x einen Wert von 0 bis 50 hat.

Eine Oxidationshaarfärbecreme (A), die für die Ausführung der Erfindung besonders geeignet ist, enthält z. B. 5 bis 15 Gew.-% Fettalkohole mit 12 bis 18 C-Atomen und 5 bis 10 Gew.-% eines Fettalkoholsulfats oder Fettalkohol(polyglycolether)sulfats der Formel I.

Eine bevorzugt geeignete Oxidationsmittelzubereitung (B) enthält z. B. 1 bis 5 Gew.-% Fettalkohole mit 12 bis 18 C-Atomen und 1 bis 10 Gew.-% eines Fettalkoholsulfats oder Fettalkoholpolyglycolethersulfats der Formel I.

Die Oxidationshaarfärbecreme (A) kann außer den genannten Komponenten noch direkt ziehende Haarfarbstoffe zur Modifikation der Nuance, z. B. vom Typ der Nitrophenylendiamine, Nitroaminophenole, Anthrachinonfarbstoffe oder Indophenole enthalten. Weiterhin können Puffersalze, z. B. Ammoniumsulfat, ein Reduktionsmittel zur Verhinderung vorzeitiger Oxidation der Oxidationsfarbstoffvorprodukte, z. B. Natriumsulfit, freier Ammoniak oder andere Basen wie z. B. Natronlauge, Mono-, Di- oder Triethanolamin zur Einstellung eines leicht alkalischen pH-Wertes von vorzugsweise 8 bis 10, Komplexbildner, z. B. Nitrilotriessigsäure oder 1-Hydroxyethan-1,1-diphosphonsäure sowie Duftstoffe und haarkosmetische Zusätze wie z. B. Proteine, Proteinderivate oder kationische Polymere in untergeordneten Mengen enthalten sein.

Die Oxidationsmittelzubereitung (B) kann außer den genannten Komponenten noch Zusätze zur Stabilisierung des Wasserstoffperoxids und zur Verhinderung vorzeitiger Zersetzung enthalten. Als solche Stabilisatoren eignen sich vor allem Stoffe, die Schwermetallionen binden, so daß sie nicht mehr als Katalysatoren für die Peroxidzersetzung fungieren. Geeignete Stabilisatoren sind z. B. Acetanilid, Phenacetin, Acetophenetidin, Kaliumhydrogenphosphat, Natriumpyrophosphat, Phosphorsäure, Citronensäure, Weinsäure, Salicylsäure, Milchsäure und/oder Dipicolinsäure, Ethylendiamintetraessigsäure, Nitrilotriessigsäure oder komplexbildende Organophosphonsäuren wie z. B. Hydroxyethandiphosphonsäure oder Nitrilotrimethylenphosphonsäure oder die wasserlöslichen Salze der genannten Säuren. In ähnlicher Weise wirken auch Korrosionsinhibitoren, die den Eintrag von Metallionen aus metallischen Behälterwandungen in die Oxidationsmittelzubereitung verhindern. Zur Korrosionsinhibierung kann z. B. Ammoniumnitrat, Natriumsilikate oder Magnesiumsilikat verwendet werden. Der pH-Wert der Oxidationsmittelzubereitung wird bevorzugt auf einen Wert zwischen 3 und 5 eingestellt.

Die Herstellung der Oxidationshaarfärbecreme (A) erfolgt in der Weise, daß die Fettkomponente und die Emulgatoren zusammen aufgeschmolzen und bei einer Temperatur von 70 bis 90 °C die Hauptmenge des auf die gleiche Temperatur erwärmten Wassers einemulgiert wird. Nach Abkühlen auf ca. 40 °C wird die wäßrige Lösung der Oxidationsfarbstoffvorprodukte, eine wäßrige Lösung der Salze, des Reduktionsmittels und des Komplexbildners und zum Schluß eine Ammoniaklösung zur Einstellung eines pH-Wertes von 8 bis 10 und das restliche Wasser zugesetzt.

Die Herstellung der Oxidationsmittelzubereitung (B) erfolgt in der Weise, daß die Fettkomponenten und die Emulgatoren zusammen aufgeschmolzen und bei einer Temperatur von 70 bis 90 °C die Hauptmenge des auf die gleiche Temperatur erwärmten Wassers einemulgiert wird. Nach dem Abkühlen auf ca. 40 °C wird die wäßrige Lösung der Salze und Stabilisatoren zugegeben. Nach dem Abkühlen auf 20 bis 30 °C wird das Wasserstoffperoxid in Form einer wäßrigen Lösung zugesetzt.

Die folgenden Beispiele sollen den Gegenstand der Erfindung näher erläutern:

## B e i s p i e l e

### Oxidationshaarfärbecreme (A)

Zusammensetzung:

| | | |
|---|---|---|
| Fettalkohol $C_{12}-C_{18}$ | 10 | Gew.-% |
| Fettalkohol $C_{16}-C_{18}$-polyglycol(20 EO)ether | 0,75 | " |
| Fettalkohol($C_{12}-C_{14}$)polyglycol(2 EO)ether-sulfat, Na-Salz, (28%ige Lösung in Wasser) | 20 | " |
| Kokosacylamidopropyldimethylammoniumglycinat (30%ige Lösung in Wasser) | 12,5 | " |
| Kationisches Cellulosederivat (Polymer JR 400) | 1 | " |
| Ethylendiamintetraessigsäure | 0,2 | " |
| Natriumsulfit | 0,5 | " |
| Ascorbinsäure | 0,5 | " |
| Ammoniumsulfat | 1,5 | " |
| Parfümöl | 0,2 | " |
| p-Toluylendiamin | 0,13 | " |
| p-Aminophenol | 0,05 | " |
| Resorcin | 0,1 | " |
| 2,4-Diaminophenetol | 0,002 | " |
| alpha-Naphtol | 0,02 | " |
| Ammoniak-Lösung bis pH = 10 | | |
| Wasser | ad 100 | " |

Die nach dieser Rezeptur hergestellte Haarfärbecreme hat eine Viskosität von 13 Pa.s bei einer Scherspannung von D = 3 sec$^{-1}$ und von 17,8 Pa.s bei einer Scherspannung von D = 6 sec$^{-1}$, gemessen bei steigender Scherspannung und bei 20 °C.
Die Viskosität wurde mit einem Haake-Rotationsviskosimeter Type RV 12/MV I gemessen.

## Oxidationsmittelzubereitung (B)

| | | |
|---|---|---|
| Cetylalkohol | 1,5 | Gew.-% |
| Fettalkohol$C_{12}$-$C_{14}$-polyglycol(2 EO)-ether | 3,0 | " |
| Fettalkohol($C_{12}$-$C_{14}$)-polyglycol(30 EO)-ether-sulfat, Na-Salz, (25%ige Lösung in Wasser) | 8,0 | " |
| Ammoniumnitrat | 0,012 | " |
| Natriumpyrophosphat | 0,03 | " |
| Dipicolinsäure | 0,10 | " |
| 1-Hydroxyethan-1,1-diphosphonsäure | 1,50 | " |
| Ammoniak-Lösung bis pH = 4,0 | | |
| Wasserstoffperoxid-Lösung (50 Gew.-% $H_2O_2$) | 12,0 | " |
| Wasser | ad 100 | " |

Die nach dieser Rezeptur hergestellte Oxidationsmittelzubereitung hat eine Viskosität von 2,35 Pa.s (D = 3 sec⁻¹) bzw. von 2,87 Pa.s (D = 6 sec⁻¹), gemessen bei steigender Scherspannung und bei 20 °C.

Gebrauchsfertige Haarfärbezubereitung (A + B)

In einem Zweikammer-Misch- und Abgabebehälter gemäß US-PS 4 247 001 wurde in die eine Kammer eine Oxidationshaarfärbecreme gemäß Beispiel 1A und in die zweite Kammer eine Oxidationsmittelzubereitung gemäß Beispiel 1B eingebracht.

Durch Druck auf den flexiblen, mit einem Schneidedorn ausgerüsteten Boden der einen Kammer wurde die Trennwand zerstört und durch Schütteln eine Durchmischung der beiden Komponenten bewirkt. Schon nach ca. 20 Sekunden Schütteln war eine homogene Mischung der Komponenten erreicht. Die dabei entstandene gebrauchsfertige Haarfärbezubereitung hatte eine Viskosität von 8,5 Pa.s (D = 3 sec⁻¹) bzw. 9,8 Pa.s (D = 6 sec⁻¹), gemessen bei steigender Scherspannung und bei 20 °C.

**Patentansprüche**

1. Oxidationshaarfärbezubereitung, bestehend aus einem Zweikammer-Misch- und Abgabebehälter und den darin verpackten Komponenten, wobei der Behälter so ausgebildet ist, daß in den zwei durch eine mechanisch zerstörbare Trennwand getrennten Kammern eine Oxidationshaarfärbecreme (A) und eine Oxidationsmittelzubereitung (B) isoliert voneinander verpackt sind und durch mechanische Zerstörung der Trennwand eine Vereinigung und Durchmischung der beiden Komponenten vor der Abgabe aus der an einer der beiden Kammern befindlichen Abgabeöffnung möglich ist, dadurch gekennzeichnet, daß
- die Oxidationshaarfärbecreme (A) in Form einer Öl-in-Wasser-Emulsion mit einem Gehalt von 0,1 bis 5,0 Gew.-% an Oxidationshaarfarbstoffvorprodukten und
- die Oxidationsmittelzubereitung (B) in Form einer Öl-in-Wasser-Emulsion mit einem Gehalt von 1,5 bis 15 Gew.-% $H_2O_2$ vorliegt
und die Komponenten A und B im Gewichtsverhältnis A : B = 3 : 1 bis 1 : 1 vorliegen und nach dem Vermischen ein Haarfärbemittel mit einer Viskosität bei 20 °C von 5 bis 15 Pa.s bei einer Scherspannung von D = 3 bis 6 sec⁻¹ ergeben.

2. Oxidationshaarfärbezubereitung nach Anspruch 1, dadurch gekennzeichnet, daß die Oxidationshaarfärbecreme (A) eine Viskosität von 5 bis 20 Pa.s und die Oxidationsmittelzubereitung (B) eine Viskosität von 0,2 bis 5 Pa.s, jeweils gemessen bei 20 °C und bei einer Scherspannung D = 3 bis 6 sec⁻¹.

3. Oxidationshaarfärbezubereitung nach den Ansprüchen 1 oder 2, dadurch gekennzeichnet, daß die Oxidationshaarfärbe creme (A) und die Oxidationsmittelzubereitung (B) als Fettkomponente einen gesättigten Fettalkohol mit 12 bis 22 C-Atomen und als Emulgator ein Alkylsulfat oder Alyklpolyglycolethersulfat der Formel R¹ - O (CH₂CH₂O)ₓ - SO₃ M enthalten, in der R¹ eine Alkylgruppe mit 8 bis 18 C-Atomen, M eine Alkali-, Magnesium-, Ammonium- oder ein Mono-, Di- oder Trialkanolammoniumion mit 2 bis 4 C-Atomen in der Alkanolgruppe ist und x einen Wert von 0 bis 50 hat.

**Claims**

1. An oxidation hair dye preparation consisting of a twochamber mixing and dispensing container and of the components packed therein, the container being designed in such a way that an oxidation hair dye cream (A) and an oxidizer preparation (B) are packed separately from one another in the chambers separated by a mechanically destructible partition and, by mechanical destruction of the partition, the two components are combined and mixed before being discharged from the outlet opening in one of the two chambers, characterized in that

– the oxidation hair dye cream (A) is in the form of an oil-in-water emulsion containing from 0.1 to 5.0% by weight oxidation hair dye precursors and

– the oxidizer preparation (B) is in the form of an oil-in-water emulsion containing from 1.5 to 15% by weight $H_2O_2$,

and in that components A and B are present in a ratio by weight of A:B of from 3:1 to 1:1 and, after mixing, give a hair dye having a viscosity at 20°C of from 5 to 15 Pa • s at a shear rate D of from 3 to 6 sec$^{-1}$.

2. An oxidation hair dye preparation as claimed in claim 1, characterized in that the oxidation hair dye cream (A) has a viscosity of from 5 to 20 Pa • s and the oxidizer preparation (B) a viscosity of from 0.2 to 5 Pa • s, measured in each case at 20°C and at a shear rate D of from 3 to 6 sec$^{-1}$.

3. An oxidation hair preparation as claimed in claim 1 or 2, characterized in that the oxidation hair dye cream (A) and the oxidizer preparation (B) contain as fatty component a saturated fatty alcohol containing from 12 to 22 C atoms and, as emulsifier, an alkyl sulfate or alkylpolyglycolether sulfate corresponding to the formula $R^1 – O\ (CH_2CH_2O)_x – SO_3M$ in which $R^3$ is a $C_6$–$C_{18}$ alkyl group, M is an alkali, magnesium or ammonium or a mono-, di- or trialkanolammonium ion containing from 2 to 4 C-atoms in the alkanol group and x has a value of from 0 to 50.

**Revendications**

1. Composition de teinture des cheveux par oxydation se composent d'un récipient à deux compartiments pour le mélange et pour la décharge, et les composants qui y sont conditionnés, pour lesquels le récipient est conçu de telle façon que dans les deux compartiments séparés par une paroi de séparation qui peut être détruite mécaniquement, une crème de teinture pour les cheveux par oxydation (A) et une composition d'agent d'oxydation (B) sont conditionnées isolément l'une de l'autre et par destruction mécanique de la paroi de séparation, une réunion et un mélange des deux composants avant la décharge par une ouverture de décharge qui se trouve sur un des deux compartiments, est possible, caractérisée en ce: la crème de teinture pour les cheveux par oxydation (A) se présente sous la forme d'une émulsion huile dans l'eau ayant une teneur allant de 0,1 à 5% en poids d'un précurseur de teinture pour les cheveux par oxydation et la composition d'agent d'oxydation (B) sous la forme d'une émulsion huile dans l'eau avec une teneur allant de 1,5 à 15% en poids d'$H_2O_2$ et les composants A et B se présentent sous un rapport pondéral A/B = 3 : 1 à 1 : 1 et après le mélange fournissent une préparation de teinture pour les cheveux ayant une viscosité à 20°C de 5 à 15 Pa • s pour une tension de cisaillement de D = 3 à 6 sec$^{-1}$.

2. Composition pour teinture des cheveux par oxydation selon la revendication 1, caractérisée en ce que la crème de teinture des cheveux par oxydation (A) présente une viscosité de 5 à 20 Pa • s et la composition d'agent d'oxydation (B) présente une viscosité allant de 0,2 à 5 Pa • s, respectivement mesurées à 20°C et pour une tension de cisaillement D = 3 à 6 sec$^{-1}$.

3. Composition de teinture pour les cheveux par oxydation selon les revendications 1 ou 2, caractérisée en ce que la crème de teinture pour les cheveux par oxydation (A) et la composition d'agent d'oxydation (B) contiennent en tant que composant gras un alcool gras saturé ayant de 12 à 22 atomes de carbone et en tant qu'émulsionnant un sulfate d'alcoyle ou un sulfate d'alcoylpolyglycoléther de formule $R_1O(CH_2CH_2O)_x–SO_3M$ dans laquelle $R_1$ est un radical alcoyle ayant de 8 à 18 atomes de carbone, M est un ion alcalin, de magnésium, d'ammonium ou de mono-, di- ou trialcanolammonium ayant de 2 à 4 atomes de carbone dans le radical alcanol et x a une valeur de 0 à 50.